# EUROPEAN PATENT APPLICATION

(11) **EP 4 559 472 A1**
(43) Date of publication of application: **28.05.2025**
(21) Application number: 23212043.6
(22) Date of filing: 24.11.2023
(51) Int. Cl.: A61K 38/17, A61P 9/10, A61P 13/12, A61P 29/00, A61P 31/00, A61P 37/02, C07K 14/47

(54) **COMPLEMENT MODULATOR**

(71) Applicant: Universität Ulm, 89081 Ulm (DE)
(72) Inventor: The designation of the inventor has not yet been filed
(74) Representative: Altmann Stößel Dick Patentanwälte PartG mbB

(57) **Abstract**

The present invention relates to a modulator protein comprising (i) a regulator module regulating the classical pathway of complement activation and/or the lectin pathway of complement activation and (ii) a binding module comprising, preferably consisting of, CCP domains 19 and 20 of a factor H; for use in preventing and/or treating a disease caused and/or aggravated by an activation of the classical and/or lectin pathway of complement activation, and to modulator proteins, methods, and kit related thereto.

## Description

The present invention relates to a modulator protein comprising (i) a regulator module regulating the classical pathway of complement activation and/or the lectin pathway of complement activation and (ii) a binding module comprising, preferably consisting of, CCP domains 19 and 20 of a factor H; for use in preventing and/or treating a disease caused and/or aggravated by an activation of the classical and/or lectin pathway of complement activation, and to modulator proteins, methods, and kits related thereto.

The innate immune system is highly conserved even in primitive organisms. The cellular effectors of this branch comprise mainly neutrophils, monocytes and macrophages, whereas the soluble innate immune effectors consist mainly of the complement system in addition to other effectors like acute phase proteins or pore-forming peptides (Parkin & Cohen (2001) The Lancet 357: 1777-89). The complement system consists of components in serum, which were described by Paul Ehrlich to "complement" the antibody response against bacteria. Some of these components are heat-labile. Other functions of the complement system are opsonisation of microbial intruders, immune complexes, debris, apoptotic and necrotic cells to support their effective clearance through uptake by phagocytic cells (Ricklin et al. (2010) Nature Immunology 11: 785-797). The complement system is organized in three activation pathways: the classical (CP), lectin (LP) and alternative pathway (AP).

Activation of the CP is typically achieved in an antibody-dependent manner via the complement component C1q, which acts as a pattern-recognition molecule (PRM). After a series of proteolytic activation events, the CP C3-convertase (C4bC2a; following the traditional nomenclature; in an attempt to harmonise the complement nomenclature some researchers propose to name the CP/LP convertase C4b2b with 2b representing the bigger cleavage fragment of C2 containing the enzymatic activity) cleaves the complement component C3, which is central to all three complement activation pathways, to C3a, an anaphylatoxin and C3b (opsonin). Due to this cleavage, a conformational change occurs and a previously internal thioester bond reaches the protein surface of C3b. This active, and once it is exposed short lived, thioester bond can bind covalently to hydroxyl- and amino-groups of molecules localized on cell surfaces, or can be hydrolysed ("quenched") by water. As a consequence, opsonisation of cells with many C3b molecules can occur if C3-convertases are not down regulated. The production of huge amounts of C3b molecules facilitates activation of C5 by C5 convertases. The C5-convertase cleaves C5 to C5a (the most potent anaphylatoxin) and C5b, which interacts with the complement factors C6-9 to form the membrane attack complex (MAC) that assembles holes in cell membranes to lyse and kill the cells.

The Lectin pathway (LP) is similarly organized as the CP. Activation occurs via recognition of pathogen-associated molecular patterns (PAMPs) or danger-associated molecular patterns (DAMPs). Within the LP, PAMPs or DAMPs can be detected by several pattern recognition molecules which are homologous to C1q (the pattern recognition molecule of the CP): mannose-binding lectin (MBL) and various types of collectins and ficolins. Subsequent to binding PAMPs or DAMPs, MBL undergoes conformational changes and then associates with MBL-associated serine proteases (MASPs). MBL is homologous in structure and function to C1q. In analogy to the CP, MASP2 proteolytically activates C2 into C2a and C2b, and C4 into C4a and C4b. The activated components can build the C3 convertase C4b2a, which is identical to the CP and cleaves C3 into C3a and C3b. In analogy to the CP, in absence of strict regulation of the C3 convertase, production of further C3b molecules fosters the activation of C5 via C5 convertases. Proteolytic activation of C5 is the starting point of the terminal and lytic complement pathway where C5b initiates formation of the MAC.

The alternative pathway (AP) gets activated through a process of self-activation at low level. This process is called "tick-over" activation of C3. C3 molecules have an intrinsically metastable conformation. At all times, a small proportion of the C3 molecules undergo spontaneous conformational changes (activation) which exposes the previously internal thioester module. The thioester can be quenched by water or attach indiscriminatingly (for self or foreign) to nucleophiles on a cell surface. Such "auto-activated" C3 is called C3(H₂O) and is structurally similar to C3b molecules. C3b or C3(H₂O) expose new protein surfaces that are hidden in C3. These new surfaces bind factor B, another complement factor of the AP. When factor B is bound to C3b or C3(H₂O), it can be cleaved by the protease factor D into Ba and Bb. Bb remains bound to C3(H₂O) (or C3b) and constitutes the C3 convertase of the AP, C3b(H₂O)Bb (or C3bBb). In analogy to the CP and LP C3 convertases, C3bBb can produce C3b and C3a molecules by cleaving C3. The protein Properdin, a positive regulator of the AP, plays an important role by stabilizing the protein-protein interactions of the AP C3 convertase.

If not regulated, any C3b generated by the alternative, classical or lectin pathway is able to build more C3 convertases of the AP and further amplify the number of produced C3b molecules in a positive feedback loop. This step is called "amplification loop" of the AP. Thus, the three pathways of activation converge at the level of C3 activation and, if not regulated, cumulate in MAC formation.

Classical and lectin pathways are inactive until they get specifically activated through the sensing of pathogens or endogenous danger molecules. The AP, on contrary, is active all the time at a low level and indiscriminately produces C3b (or initially C3(H₂O)) molecules. More than ten different regulatory proteins within the complement system are known. Some regulators inhibit right at the level of initiating the CP and LP, however the parts of the cascade that are most tightly controlled are the convertases, which act as amplifiers of the activation signal, and C3b, which builds the platform to form the C3-, and the inflammatory C5-convertases. There are also some regulators that specifically control the lytic MAC.

Regulatory proteins can be divided into decay accelerators which destabilize C3-convertases and lead to faster decay of the convertases. A further group involves proteins which degrade C3b or/and C4b. This is achieved by factor I which however needs a cofactor in order to cleave C3b and/or C4b; the cofactor is needed to prevent non-specific degradation by the soluble protease factor I. Thus, inactivation of C3b or C4b necessitates the presence of cofactor proteins that bind to the target and recruit factor I (e.g. factor H or CR1 or C4b-binding protein (C4BP)). A further group of regulators inhibits formation of MAC.

Complement control protein (CCP) domains are peptide sequences comprising approx. 60 to 70 amino acids including a conserved tryptophan and four conserved cysteine residues forming two disulfide bonds, with considerable sequence variation of the residual amino acids. Besides binding to complement proteins C3b and/or C4b, CCP domains were found to mediate further activities, including decay accelerating activity and factor I cofactor activity. CCP domains were e.g. reviewed in Schmidt et al. (2008), Clin Exp Immunol. 151(1): 14-24.

Many diseases, in particular hereditary diseases, are associated with a malfunction of complement, in particular overactivation of complement. Thus, in an effort to provide an artificial regulator of the complement system, a monoclonal antibody specifically binding to complement protein C5 and inhibiting terminal activation, eculizumab, was developed (Hillmen et al. (2006), NEJM355(12):1233). In a similar line of development, C5 inhibitory protein rEV576 (coversin) was developed (Romay-Penabad et al (2014), Lupus 23(12):1324). Further, a protein called "mini-FH", connecting complement control protein repeats (CCP domains) 1-4 and 19-20 of complement factor H via a linker was obtained (WO 2013/142362 A1). MiniFH was described to have an increased complement regulatory activity and to outperform FH in its regulatory activity directed towards the complement alternative pathway tenfold in several *in vitro* and *ex vivo* assays.

Nonetheless, there is still a need in the art for improved complement inhibitors avoiding the drawbacks of the prior art. This problem is solved by the means and methods disclosed herein.

In accordance, the present invention relates to a modulator protein comprising (i) a regulator module regulating the classical pathway of complement activation and/or the lectin pathway of complement activation and (ii) a binding module comprising, preferably consisting of, CCP domains 19 and 20 of a factor H.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a multitude of cells. Also, the term "plurality" relates to a multitude, preferably at least two, more preferably at least three, still more preferably at least four, of the indicated items.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are *in vitro* methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

As used herein, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s). As referred to herein, in case of doubt, database entries relate to the entry current at the time of filing of the instant application.

The term "polynucleotide", as used herein, refers to a linear or circular nucleic acid molecule. The polynucleotide of the present invention shall be provided, preferably, either as an isolated polynucleotide (i.e. isolated from its natural context) or in genetically modified form, preferably comprising at least one heterologous sequence. The term polynucleotide encompasses singleas well as, partially or completely, double-stranded polynucleotides. Preferably, the polynucleotide is a DNA polynucleotide, which may also be referred to as "DNA". Moreover, comprised are also chemically modified polynucleotides including naturally occurring modified polynucleotides such as glycosylated or methylated polynucleotides or artificially modified derivatives such as biotinylated polynucleotides, locked nucleic acids, peptide nucleic acids, and the like. In view of the description herein, template DNA, e.g. for PCR, primers, e.g. sequencing or amplification primers, and probe oligonucleotides are included in the term polynucleotides.

Unless specifically indicated otherwise, reference to specific polynucleotides herein preferably includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a polynucleotide may e.g. be an ortholog, a paralog, or another homolog of the specific polynucleotide; the polynucleotide variant may also be a mutant of the specific polynucleotide, preferably a naturally occurring mutant, e.g. identified in a cancer cell. Also preferably, said polynucleotide variant is or is derived from a non-naturally occurring allele of the specific polynucleotide. Further polynucleotide variants include polynucleotides comprising nucleic acid sequences which are at least 70%, preferably at least 80%, more preferably at least 90%, even more preferably at least 95%, still more preferably at least 98%, most preferably at least 99%, identical to the specifically indicated nucleic acid sequences and/or encoding polypeptides having amino acid sequences which are at least 70%, preferably at least 80%, more preferably at least 90%, even more preferably at least 95%, still more preferably at least 98%, most preferably at least 99%, identical to the specifically indicated amino acid sequences. The percent identity values are, preferably, calculated over the entire nucleic acid sequence region, preferably as specified herein elsewhere. The polynucleotides of the present invention either consist, essentially consist of, or comprise the aforementioned nucleic acid sequences. Thus, they may contain further nucleic acid sequences as well.

The term "protein" is understood by the skilled person; preferably, the protein comprises at least one amino acid chain, i.e. a polypeptide as specified herein below, more preferably comprises a multitude of polypeptides. Thus, the protein may be a multimer, e.g. a dimer, a trimer, or the like, wherein the polypeptides in the multimer may be connected covalently, e.g. by a disulfide bridge, or non-covalently, e.g. by ionic interactions, hydrophobic interactions, and/or van der Waals interactions. The protein may consist of identical polypeptides, e.g. may be a homodimer, or may comprise at least two non-identical polypeptides, e.g. may be a heterodimer. More preferably, the protein as specified comprises all structural components as indicated comprised in one continuous covalent polypeptide chain, thus, the protein preferably is or is comprised in a polypeptide, e.g. a fusion polypeptide.

The term "polypeptide", as used herein, refers to a molecule comprising of a multitude of amino acids that are covalently linked to each other by peptide bonds. Polypeptides consisting of less than 20 amino acids covalently linked by peptide bonds may also be referred to as "peptides". Preferably, the polypeptide comprises of from 100 to 1000, more preferably of from 150 to 1000, still more preferably of from 200 to 500, most preferably of from 250 to 400 amino acids. The polypeptide may also be comprised in a fusion polypeptide, i.e. may comprise amino acid sequences in addition to those specifically indicated. Also, the polypeptide may comprise additional, non-peptidic structures, such as at least one glycosylation, lipid conjugation, and the like. Thus, unless specifically indicated otherwise, reference to specific polypeptides herein preferably includes polypeptide variants.

As used herein, the term "polypeptide variant" relates to any chemical molecule comprising at least one polypeptide as specified herein differing in structure from a specifically indicated polypeptide. Preferably, the polypeptide variant comprises a polypeptide having a contiguous amino acid sequence corresponding to at least 70%, preferably at least 80%, more preferably at least 90%, even more preferably at least 95%, still more preferably at least 98%, most preferably at least 99%, of the amino acid sequence of the polypeptide specifically indicated. Moreover, it is to be understood that a polypeptide variant as referred to in accordance with the present invention shall have an amino acid sequence which differs due to at least one amino acid substitution, deletion and/or addition, wherein the amino acid sequence of the variant is still, preferably, at least 70%, more preferably at least 80%, even more preferably at least 90%, even more preferably at least 95%, still more preferably at least 98%, most preferably at least 99%, identical with the amino acid sequence of the specific polypeptide. The degree of identity between two amino acid sequences can be determined by algorithms well known in the art and as described herein elsewhere. Polypeptide variants referred to above may be allelic variants or any other species specific homologs, paralogs, or orthologs. Moreover, the polypeptide variants referred to herein include fragments of the specific polypeptides or the aforementioned types of polypeptide variants, preferably as long as these fragments and/or variants have the activity as specified. Such fragments may be or may be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics. The "activity" of a polypeptide as specified herein is, preferably, preserved in the polypeptide variant; as the skilled person will understand in view of the description herein, the activity of a polypeptide does not necessarily have to reflect its main natural function, but may be a further activity relevant in the context of the claimed invention. On a strictly exemplary basis, the p53 polypeptide has an activity in cell cycle regulation, but p53 and in particular mutants thereof also are immunogenic. Thus, a p53 mutant, including a fragment thereof, is a p53 variant as referred to herein, preferably having the activity of being immunogenic. The above applies to peptide variants and to protein variants *mutatis mutandis.*

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or activity. Thus, the term includes sub-parts generated by actual fragmentation of a biological macromolecule, but also sub-parts derived from the respective biological macromolecule in an abstract manner, e.g. *in silico.* Thus, as used herein, an Fc or Fab fragment, but also e.g. a singlechain antibody, a bispecific antibody, and a nanobody may be referred to as fragments of an immunoglobulin. Also, a disease epitope may be a fragment of a disease antigen.

Unless specifically indicated otherwise herein, the compounds specified, in particular the polynucleotides and polypeptides, may be comprised in larger structures, e.g. may be covalently or non-covalently linked to further sequences, adjuvants, carrier molecules, retardants, and other excipients. In particular, polypeptides as specified may be comprised in fusion polypeptides comprising further polypeptides, which may serve e.g. as a tag for purification and/or detection, as a linker, or to extend the *in vivo* half-life of a compound. The term "detectable tag" refers to a stretch of amino acids which are added to or introduced into the fusion polypeptide; preferably, the tag is added C- or N- terminally to the fusion polypeptide. Said stretch of amino acids preferably allows for detection of the polypeptide by an antibody which specifically recognizes the tag; or it preferably allows for forming a functional conformation, such as a chelator; or it preferably allows for visualization, e.g. in the case of fluorescent tags. Preferred detectable tags are the Myc-tag, FLAG-tag, 6-His-tag, HA-tag, GST-tag or a fluorescent protein tag, e.g. a GFP-tag. These tags are all well known in the art. Other further peptides preferably comprised in a fusion polypeptide comprise further amino acids or other modifications which may serve as mediators of secretion, as mediators of blood-brain-barrier passage, as cell-penetrating peptides, and/or as immune stimulants. Further polypeptides or peptides to which the polypeptides may be fused are signal and/or transport sequences, e.g. an IL-2 signal sequence, and linker sequences.

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide as specified herein, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (e.g. BLAST, GAP, BESTFIT, PASTA, or TFASTA), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. More preferably, the Basic Local Alignment Search Tool (BLAST) implementation is used with default parameter values for alignment. In the context of biological sequences referred to herein, the term "essentially identical" indicates a %identity value of at least 90%, preferably at least 95%, more preferably at least 98%, most preferably at least 99%. As will be understood, the term essentially identical includes 100% identity. The aforesaid applies to the term "essentially complementary" *mutatis mutandis.*

The term "complement control protein repeat domain", which may also be referred to as "complement control protein repeat", "CCP domain", or "CCP" herein and which is also known as "short complement-like repeat", "short consensus repeat" or "SCR" in the art, is, in principle, known to the skilled person, as described herein above. CCP domains having a specified activity are known in the art and are described in the context of the corresponding activity.

The three major pathways of complement activation, i.e. the classical pathway (CP), the lectin pathway (LP), and the alternative pathway (AP), have been known in the art for a long time and have been described herein above.

The term "factor H" is known to the skilled person as a cofactor in the inactivation of C3b by factor I and for increasing the rate of dissociation of the C3bBb complex (C3 convertase) and the C3bBb3b complex (C5 convertase) in the alternative complement pathway. Besides these complement regulatory functions, CCP domains of factor H or stretches thereof may have other biological functions that augment the complement regulatory functions in vivo, like glycosaminoglycan binding, binding to sialic acid or C3 activation/inactivation fragments like C3b, iC3b C3dg and C3d. Preferably, factor H is a mammalian factor H, more preferably human factor H. Most preferably, factor H is human factor H having an amino acid sequence as specified in Genbank Acc. No. NP_000177.2, Uniprot Acc No. P08603 (CFAH_HUMAN). CCP domains 19-20 of factor H preferably comprise, preferably consist of, the amino acid sequence as shown in SEQ ID NO:1 or a sequence at least 70% identical thereto. Also preferably, CCP domains 19-20 of factor H comprise, preferably consist of, the amino acid sequence as shown in SEQ ID NO:2 or a sequence at least 70% identical thereto.

The term "C4b binding protein", which may be abbreviated as "C4BP" is known to the skilled person as a control molecule of the classical/lectin pathway of complement activation binding as a cofactor to C4b, and weakly to C3b, for proteolytic inactivation of the complement protein C4b (and C3b) by the serum protease factor I. C4BP also increases the rate of dissociation of the C4b2a complex (C3 convertase) and the C4b2a3b complex (C5 convertase) in the classical/lectin complement pathway. Preferably, C4BP is mammalian C4BP, more preferably human C4BP. Most preferably, C4BP is human C4BP having an amino acid sequence as specified in Genbank Acc. No: AAA36507.1 (alpha chain, Uniprot Acc. No. P04003 (C4BPA_HUMAN)); and/or Genbank. Acc. No. AAA35615.1 (beta chain, Uniprot Acc. No. P20851 (C4BPB_HUMAN)).

The term "regulating" is known to the skilled person. Preferably, the term relates to any influence exerted onto a system causing a change of at least one target parameter to a desired value. Thus, regulating complement activation includes each and every activity causing a complement activation state to change, i.e. a modulation of complement activation. The modulation may be an activation, i.e. lead to an enhanced activation; or the modulation may be an inhibition, i.e. lead to a decreased activation. The modulation preferably is an inhibition, i.e. complement activation preferably is decreased by the regulating as referred to herein.

The term "regulator module", as used herein, relates to a module, i.e. a substructure, of the modulator protein comprising a polypeptide having the activity of mediating regulation of the classical and/or lectin pathways of complement activation. Regulator modules regulating the classical pathway of complement activation and/or the lectin pathway of complement activation are known in the art and are described herein elsewhere. Preferably, the regulator module does not regulate, in particular does not inhibit, the alternative pathway of complement activation. More preferably, the modulator protein does not regulate, in particular does not inhibit, the alternative pathway of complement activation.

Preferably, the regulator module comprises a module of an effector protein of the classical and/or lectin pathway of complement activation, preferably a convertase decay accelerating module and/or a convertase, preferably C4b-based or C4b- and C3b-based, specific cofactor module. Thus, the regulator module preferably comprises at least one, preferably at least three, more preferably at least four, CCP(s), more preferably having convertase decay acceleration activity for convertases of the classical and/or lectin pathways of complement activation, wherein the term "decay accelerating activity" preferably relates to the property of a module, such as a CCP domain or a CCP domain stretch, to mediate decay, preferably inactivation, of the C3 convertase of the classical and lectin pathways of complement activation, i.e. C4bC2a. Alternatively, the regulatory property of a module, such as a CCP domain or a CCP domain stretch, may be to mediate cofactor activity for factor I mediated degradation of C4b or C4b and C3b. Another possibility is that the regulatory property of a module, such as a CCP domain or a CCP domain stretch, simultaneously contains the activities to mediate decay acceleration and cofactor activity towards the classical and lectin pathway, or the classical, lectin and alternative pathway.

Preferably, decay accelerating activity of a regulator module is determined by surface plasmon resonance (SPR). Preferably, the regulator module comprises, preferably consists of, an effector module of a C4b binding protein, of a decay accelerating protein (CD55), of a membrane cofactor protein (CD46), of factor H and/or of a complement receptor 1 (CR1; CD35, preferably comprising the amino acid sequence of SEQ ID NO:10, 11, and/or 12), wherein the term "effector module" relates to a module, e.g. a domain, of a protein mediating the indicated effect. Thus, the effector module preferably is a substructure of the indicated protein having the activity of regulating the classical and/or lectin pathway of complement activation. Preferably, the aforesaid C4b binding protein is C4b binding protein, the aforesaid decay accelerating protein is CD55, the aforesaid membrane cofactor protein is CD46, and/or the aforesaid CR is CR1. Preferably, effector module of a C4b binding protein comprises CCP domains 1-4 of the alpha chain; also preferably the effector module of a decay accelerating factor protein (CD55) comprises CCP domains 1-4, preferably comprising the amino acid sequence of SEQ ID NO:8 or a sequence at least 70% identical thereto; also preferably the effector module of a membrane cofactor protein (CD 46) comprises CCP domains 1-4, preferably comprising the amino acid sequence of SEQ ID NO:9 or a sequence at least 70% identical thereto; and also preferably the effector module of a complement receptor 1 (CR1) comprises CCP domains 1-3, 8-10, 15-17, or a combination thereof; preferably comprising the amino acid sequence of SEQ ID NO:10, 11, and/or 12 or a sequence at least 70% identical thereto.

Preferably, the regulator module comprises CCP domains 1 to 4 of C4BP, i.e. preferably comprises the amino acid sequence of SEQ ID NO:3 or a sequence at least 70% identical thereto, more preferably consists of the amino acid sequence of SEQ ID NO:3 or a sequence at least 70% identical thereto. Also preferably, the regulator module comprises the amino acid sequence of SEQ ID NO:3, more preferably consists of the amino acid sequence of SEQ ID NO:3.

The term "binding module", as referred to herein, relates to a module of the modulator protein comprising CCP domains 19 and 20 of a factor H. Preferably, in the aforesaid amino acid sequence of said binding module the amino acid sequence aspartate at position 1119 was exchanged for a glycine. Thus, the binding module preferably comprises the amino acid sequence of SEQ ID NO:1 or 2 or a sequence at least 70% identical thereto, more preferably consists of the amino acid sequence of SEQ ID NO: 1 or 2 or a sequence at least 70% identical thereto. Also preferably, the binding module comprises the amino acid sequence of SEQ ID NO:1 or 2, more preferably binding module consists of the amino acid sequence of SEQ ID NO: 1 or 2. As described in more detail herein in the Examples, the binding module as specified was surprisingly found to have the activity of binding to complement factor C4b and to its proteolysis derivative C4d. Thus, the binding module preferably mediates binding to at least one of a C4b polypeptide, an iC4b polypeptide, a C4dg polypeptide, and a C4d polypeptide.

As used herein, the term "modulator protein" relates to any chemical molecule comprising at least the polypeptide modules as specified. It is to be understood that the chemical linkage between the modules need not necessarily be a peptide bond. It is also envisaged by the present invention that the chemical bond between the modules is an ester bond, a disulfide bond, or any other suitable covalent chemical bond known to the skilled artisan. Also envisaged are noncovalent bonds with a dissociation constant so low that a module will only dissociate to a negligible extent from the other modules. Preferably, the dissociation constant for said noncovalent bond is less than 10⁻⁵ M (as it is the case with the Strep-Tag : Strep-Tactin binding), less than 10⁻⁶ M (as it is the case in the Strep-TagII : Strep-Tactin binding), less than 10⁻8 M, less than 10⁻¹⁰ M, or less than 10⁻¹² M (as it is the case for the Streptavidin : Biotin binding). Methods of determining dissociation constants are well known to the skilled artisan and include, e.g., spectroscopic titration methods, surface plasmon resonance measurements, equilibrium dialysis and the like. Moreover, it is also envisaged that the binding between the modules of the modulator protein is indirect via an affinity pair, e.g. that the binding module comprises a tag with affinity for biotin and the regulating module is covalently coupled to biotin; or that one of the modules is coupled to a bacteriocin, while the second module is coupled to the corresponding immunity protein. Preferably, however, the chemical linkage between the modules is a peptide bond, i.e., preferably, the modulator protein is a modulator polypeptide comprising or consisting of the modules described. Preferably, the modulator protein consists of the components as described herein, preferably as a fusion polypeptide.

The modulator protein may also comprise further modules such as polypeptides; such a further module may e.g. be a module inactivating or regulating the alternative or the classical and lectin pathway of complement activation or all complement activating pathways together, modules increasing serum stability, and the like. Corresponding modules are known in the art.

The modulator protein may comprise the indicated modules in any order deemed appropriate by the skilled person; however, in particular in case the modulator protein is a modulator polypeptide, the order is the order as indicated. Thus, the modulator polypeptide preferably comprises the modules in the order N-terminus - regulator module - binding module - C-terminus. Preferably, at least two modules of the modulator protein are connected by a "linker" peptide. Suitable linker peptides are, in principle, known in the art. Preferred linker peptides comprise or, preferably, consist of glycine, alanine, and/or proline residues. More preferably, a linker peptide is a poly-glycine linker peptide. Most preferably, a linker peptide is a linker comprising, preferably consisting of, 5 to 15 glycine residues. Preferably, it is also envisaged that two modules are connected by exchanging the last amino acid of the N-terminal module and/or the first amino acid of the C-terminal module for a G residue. Preferably the modulator protein comprises the amino acid sequence of SEQ ID NOs:4 or 5, or a sequence at least 70% identical thereto, more preferably consists of the amino acid sequence of any one of SEQ ID NOs:4 or 5, or a sequence at least 70% identical thereto. Also preferably, the modulator protein comprises the amino acid sequence of any one of SEQ ID NO:4 or 5, more preferably consists of the amino acid sequence of SEQ ID NO:4 or 5.

The modulator protein referred to herein has two activities as determined by its two modules, i.e. a regulating activity as conferred by the regulating module, and a binding activity as conferred by the binding module. As the skilled person understands, regulating activity of the regulatory module preferably requires binding of the regulatory module to its regulated target, e.g. C4BP binds to C4b to regulate. Thus, the modulator protein preferably has the activity of regulating, preferably inhibiting, the classical pathway of complement activation and/or the lectin pathway of complement activation, and has the activity of binding to a C4b polypeptide, an iC4b polypeptide a C4dg polypeptide, and/or a C4d polypeptide, preferably when said polypeptide(s) is/are bound to a cell surface. Since C4b/d are deposited on the surface of cells, with C4b molecules marking them as targets for the membrane attack complex (MAC) of the complement system, the modulator protein preferably has the activity of protecting cells from detrimental effects of complement activation, preferably *in vitro* and/or *in vivo.* Preferably, the modulator protein has the activity of inhibiting the activation of the classical pathway and the lectin pathway of complement activation. Also preferably, the modulator protein comprises a module, preferably an additional module non-identical to the regulator module, wherein said module inhibits activation of the alternative pathway of complement activation; in such case, the modulator protein preferably has the activity of inhibiting the alternative pathway, the classical pathway, and the lectin pathway of complement activation.

Also preferably, a host surface of a subject is any inner surface present in a subject, wherein said inner surface may be natural or artificial. Artificial surfaces include in particular surfaces of implants. Natural surfaces are known to the skilled person and include in particular any surface of an inner lumen of a subject, such as an inner surface of a blood vessel, a mucous membrane, glomerular basement membrane, Bruch's membrane, proteoglycans, glycans, extracellular matrix, cell or other surfaces bordering the interstitium and/or the layers of the skin.

The modulator protein preferably binds to cells and/or host surfaces on which the classical pathway and/or the lectin pathway of complement activation was activated, wherein the term "cells and/or host surfaces on which the classical pathway and/or the lectin pathway of complement activation was activated" relates to cells and/or host surfaces which have attached to their surface at least one of a C4b polypeptide, an iC4b polypeptide, a C4dg polypeptide, and a C4d polypeptide. Preferably, said cells are neurons, muscle cells, endothelial cells, cells in the kidney, endothelial cells, blood cells, or epithelial cells. Preferably, said cells in the kidney are cells in a glomerulus or tubulus. Also preferably, a host surface of a subject is any inner surface present in a subject, wherein said inner surface may be natural or artificial. Artificial surfaces include in particular surfaces of implants. Natural surfaces are known to the skilled person and include in particular any surface of an inner lumen of a subject, such as an inner surface of a blood vessel, a mucous membrane, a glomerular basement membrane, a Bruch's membrane, a surface comprising at least one of a proteoglycan, a glycan, an extracellular matrix, or another surface, in particular bordering the interstitium and/or the layers of the skin. Also preferably, said endothelial cells or other host surfaces include cells and/or host surfaces in the vasculature of the eye, e.g. in the choroid, including Bruch's membrane. Also preferably, the blood cells are erythrocytes, platelets, or leukocytes.

Advantageously and surprisingly, it was found in the work underlying the present invention that a domain stretch of factor H, which is a specific regulator of the alternative pathway of complement activation, has binding activity to C4b and C4d, which are products of the classical and lectin pathways. Thus, it was newly and surprisingly found that factor H, and in particular its CCP domains 19 and 20, can be used to protect cells from the effects of activation of the classical and/or lectin pathway(s) of complement activation.

The definitions made above apply *mutatis mutandis* to the following. Additional definitions and explanations made further below also apply for all embodiments described in this specification *mutatis mutandis.*

Preferably, the modulator protein is comprised in a pharmaceutical composition. Thus, the present invention also relates to a pharmaceutical composition comprising the modulator protein described herein.

The terms "medicament" and "pharmaceutical composition" are used essentially interchangeably herein and are, in principle, known to the skilled person. As referred to herein, the terms preferably relate to any composition of matter comprising the specified active agent(s) as pharmaceutically active compound(s) and, optionally, one or more excipient. The pharmaceutically active compound(s) can be present in liquid or dry, e.g. lyophilized, form. It will be appreciated that the form and character of the pharmaceutical acceptable excipient, e.g. carrier or diluent, is dictated by the amount of active ingredient with which it is to be combined, the route of administration, and other well-known variables. The excipient(s) must be acceptable in the sense of being compatible with the other ingredients of the formulation and being not deleterious to the recipient thereof. The excipient employed may include a solid, a gel, or a liquid. Exemplary of solid carriers are lactose, terra alba, sucrose, talc, gelatin, agar, pectin, acacia, magnesium stearate, stearic acid, and the like. Exemplary of liquid carriers are phosphate buffered saline solution, physiological saline, Ringer's solutions, dextrose solution, Hank's solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The excipient(s) is/are selected so as not to affect the biological activity of the combination.

The medicament may be administered by any route deemed appropriate, e.g. by a medical practitioner, preferably at a therapeutically effective dose. The medicament is, preferably, administered topically or, more preferably, systemically. Suitable routes of administration conventionally used for drug administration are topical, intravenous, or parenteral administration as well as inhalation. Preferably, administration is systemic, more preferably intravenously. However, depending on the nature and mode of action of the specific compound(s) administered and on the clinical situation, the pharmaceutical composition may be administered by other routes as well. Moreover, the pharmaceutical composition can be administered in combination with other further active compounds either in a common pharmaceutical composition or as separated pharmaceutical compositions wherein said separated pharmaceutical compositions may be provided in form of a kit of parts.

A therapeutically effective dose refers to an amount of the active compound which prevents, ameliorates or cures the symptoms accompanying a disease or condition referred to in this specification. Therapeutic efficacy and toxicity of a drug can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and by clinical factors. As is well known in the medical arts, dosages for any one patient may depend upon many factors, including type and severity of disease, the patient's size, age, the particular formulation of the medicament to be administered, sex, time and route of administration, general health, and other drugs being administered concurrently. The medicament referred to herein is, preferably, administered at least once, e.g. as a bolus. However, the medicament may be administered more than one time and, preferably, at least twice, e.g. permanently or periodically after defined time windows. Progress can be monitored by periodic assessment. Dosage recommendations may be indicated in the prescriber or user instructions in order to anticipate dose adjustments depending on the considered recipient. The medicament according to the present invention may comprise further active agents in addition to the aforementioned active agent(s). Also, it is to be understood that the formulation of a pharmaceutical composition preferably takes place under GMP standardized conditions or the like in order to ensure quality, pharmaceutical safety, and effectiveness of the medicament.

The present invention furthermore relates to a polynucleotide encoding the modulator protein described herein, preferably encoding a modulator polypeptide described herein.

The present invention further relates to a modulator protein described herein elsewhere, the pharmaceutical composition described herein elsewhere, and/or the polynucleotide described herein elsewhere, for use in medicine; and to a use of the modulator protein described herein for the manufacture of a medicament.

The present invention also relates to a modulator protein described herein, a pharmaceutical composition described herein, and/or a polynucleotide described herein, for use in preventing and/or treating cell or host surface damage by components of the classical pathway and/or lectin pathway of complement activation.

Also, the present invention relates to a modulator protein described herein, a pharmaceutical composition described herein, and/or a polynucleotide described herein, for use in preventing and/or treating a disease caused and/or aggravated by an activation of the classical and/or lectin-dependent pathway of complement activation. Also, the present invention relates to a use of the modulator protein described herein for the manufacture of a medicament for treating and/or preventing cell or host surface damage by components of the classical and/or lectin pathway of complement activation and/or for preventing and/or treating a disease caused and/or aggravated by an activation of the classical and/or lectin pathway of complement activation.

Thus, the present invention relates to a modulator protein comprising (i) a regulator module regulating the classical pathway of complement activation and/or the lectin pathway of complement activation and (ii) a binding module comprising, preferably consisting of, CCP domains 19 and 20 of a factor H; for use in preventing and/or treating a disease caused and/or aggravated by an activation of the classical and/or lectin pathway of complement activation.

The term "disease caused and/or aggravated by an activation of the classical and/or lectin pathway of complement activation" is understood by the skilled person; corresponding diseases have been reviewed e.g. by Ricklin et al (2017), Mol Immunol. 89:10-21. Preferably, the term includes each and every disease in which activation of the classical and/or lectin pathway of complement activation contributes to severity of at least one of the diseases symptoms. Preferably, said disease is a disease in which complement activation via the classical and/or disease pathway causes damage to cells and/or at least one host surface of a subject. Preferably, said cells of a subject are cells accessible to the blood of a subject, e.g. cells lining blood vessels. Also preferably, a host surface of a subject is any inner surface present in a subject, wherein said inner surface may be natural or artificial. Artificial surfaces include in particular surfaces of implants. Natural surfaces are known to the skilled person and include in particular any surface of an inner lumen of a subject, preferably as specified herein above. Thus, the host surface may in particular be a basement membrane or a glycocalyx, e.g. of a blood vessel, a glomerulus, or a tubulus. Thus, the aforesaid cell or host surface damage may be tissue damage and/or organ damage. The aforesaid damage caused by complement activation via the classical and/or lectin pathway preferably comprises, more preferably is characterised by, deposition of a C4b polypeptide, an iC4b polypeptide, a C4dg polypeptide, and/or a C4d polypeptide on said cells or host surface. Methods for determining such deposition are known in the art. In view of the above, the disease preferably is an infection by a pathogenic microorganism, systemic lupus erythematosus, lupus nephritis, antiphospholipid syndrome, catastrophic antiphospholipid syndrome, platelet transfusion refractoriness, IgA nephropathy, transplant-associated microangiopathy, hematopoetic stem cell transplantation-associated microangiopathy, antibody-mediated rejection, ischemia-reperfusion injury, secondary membranous nephropathy, xenotransplantation, acute kidney injury, or Guillain-Barre syndrome.

Also preferably, the disease is selected from the list consisting of adhesive capsulitis, agerelated macular degeneration, Alzheimer's disease, amyotrophic lateral sclerosis, anaphylaxis, argyrophilic grain dementia, arthritis (e.g., rheumatoid arthritis), asthma, atherosclerosis, atrial fibrosis, atypical hemolytic uremic syndrome, anterior subcap sular cataract, autoimmune diseases (including, e.g., autoimmune hemolytic anemia (AIHA) ; warm AIHA ; mixed AIHA; Barraquer-Simons syndrome, Behçget's disease, British type amyloid angiopathy, bronchiectasis , bullous pemphigoid, Buerger's disease, C3 glomerulopathy and other glomerulonephritides, chronic kidney disease, Clq nephropathy, cancer, catastrophic antiphospholipid syndrome, cirrhosis, cerebral amyloid angiopathy, cold agglutinin disease, corticobasal degeneration, Creutzfeldt - Jakob disease, Crohn's disease, cryoglobulinemic vasculitis, cystic fibrosis, dementia pugilistica, dementia with Lewy Bodies (DLB), diffuse neurofibrillary tangles with calcification, Discoid lupus erythematosus, Down's syndrome, Dupuytren's contracture, Evan's syndrome, endometriosis, endomyocardial fibrosis, excessive wound healing, focal segmental glomerulosclerosis, formal thought disorder, frontotemporal dementia (FTD), frontotemporal dementia with parkinsonism linked to chromosome 17, frontotemporal lobar degeneration, Gerstmann - Straussler - Scheinker disease, Guillain - Barré syndrome, Hallervorden - Spatz disease, hemolytic - uremic syndrome, hereditary angioedema, hypophosphastasis, IgA nephropathy, idiopathic pulmonary fibrosis, idiopathic pneumonia syndrome, immune complex diseases, inclusion body myositis, infectious disease, inflammatory disease, ischemia /reperfusion injury, keloids, mild cognitive impairment, immunothrombocytopenic purpura (ITP), molybdenum cofactor deficiency (MoCD) type A, membranoproliferative glomerulonephritis (MPGN) I, membranoproliferative glomerulonephritis (MPGN) II (dense deposit disease), membranous nephritis, multi - infarct dementia, lupus (e.g., systemic lupus erythematosus (SLE)), glomerulonephritis, Kawasaki disease, multifocal motor neuropathy, multiple sclerosis, multiple system atrophy, myasthenia gravis, myocardial infarction, myotonic dystrophy, myelofibrosis, neuromyelitis optica, Niemann Pick disease type C, nonalcoholic fatty liver disease, non - Guamanian motor neuron disease with neurofibrillary tangles, Parkinson's disease, Parkinson's disease with dementia, paroxysmal nocturnal hemoglobinuria, Pemphigus vulgaris, Peyronie's disease, Pick's disease, postencephalitic parkinsonism, polymyositis, prion protein cerebral amyloid angiopathy, progressive subcortical gliosis, progressive supranuclear palsy, posterior capsule opacification, psoriasis, sepsis, Shiga toxin E. coli (STEC) -Hus, spinal muscular atrophy, stroke, subacute sclerosing panencephalitis, scleroderma, systemic sclerosis, Tangle only dementia, transplant rejection, vasculitis (e.g., ANCA associated vasculitis), Wegner's granulomatosis, sickle cell disease, cryoglobulinemia, mixed cryoglobulinemia, essential mixed cryoglobulinemia, Type II mixed cryoglobulinemia, Type III mixed cryoglobulinemia, nephritis, drug - induced thrombocytopenia, lupus nephritis, Epidermolysis bullosa acquisita, delayed hemolytic transfusion reaction, hypocomplementemic urticarial vasculitis syndrome, pseudophakic bullous keratopathy, valvular fibrosis, and platelet refractoriness.

The term "subject", as used herein, relates to an animal having a complement system, preferably to a mammal. More preferably, the subject is cattle, a pig, sheep, horse, cat, dog, mouse, monkey, or rat, most preferably a human. Preferably, the subject is known or suspected to suffer from a disease caused and/or aggravated by an activation of the classical and/or lectin pathway of complement activation, or it is known or suspected that the subject will develop such disease within the next 6 months, preferably 4 weeks, more preferably 7 days, most preferably 24 hours.

The terms "treating" and "treatment" refer to an amelioration of a disease or disorder referred to herein or the symptoms accompanied therewith to a significant extent; as used herein, the term includes prevention of deterioration of a disease, disorder, or symptoms associated therewith. Said treating may also include an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50% at least 60%, at least 70%, at least 80%, or at least 90% of the subjects of a given cohort or population.

The terms "preventing" and "prevention" refer to retaining health with respect to the diseases or disorders referred to herein for a certain period of time in a subject. It will be understood that the said period of time may be dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

In the context of the treatments and preventions specified herein, the regulator module preferably further regulates the alternative pathway of complement activation. More preferably, in said context, the modulator protein further regulates, in particular inhibits, the alternative pathway of complement activation.

Thus, the present invention also relates to a modulator protein comprising (i) a regulator module regulating the classical pathway of complement activation and/or the lectin pathway of complement activation, and optionally the alternative pathway of complement activation; and (ii) a binding module comprising, preferably consisting of, CCP domains 19 and 20 of a factor H; for use in preventing and/or treating a disease caused and/or aggravated by an activation of the classical and/or lectin pathway of complement activation.

The present invention also relates to a method for treating and/or preventing cell damage by components of the classical and/or lectin pathway of complement activation and/or for preventing and/or treating a disease caused and/or aggravated by an activation of the classical and/or lectin-dependent pathway of complement activation in a subject, said method comprising
(a) administering a modulator protein as described herein to said subject; and
(b) thereby treating and/or preventing cell and/or host surface damage by components of the classical and/or lectin pathway of complement activation and/or for preventing and/or treating a disease caused and/or aggravated by an activation of the classical and/or lectin-dependent pathway of complement activation in said subject.

The method for treating and/or preventing of the present invention, preferably, is an *in vivo* method; the method or steps thereof may, however, also be performed *in vitro,* e.g. by pretreating an organ graft according to step (a) before implantation. As the skilled person understands, a modulator protein may be administered to an organ graft by (i) administering the modulator protein to the donor subject before explantation, (ii) by perfusing the ogran graft with the modulator protein in vitro, preferably after explantation from the donor subject and before implantation into the recipient subject, and/or (iii) by administering the modulator protein to the recipient subject, preferably before, concomitant, and/or after transplantation.

The present invention further relates to a method, preferably an *in vitro* method, for preventing damage by components of the classical and/or lectin pathway of complement activation to a host cell and/or surface, said method comprising
(A) contacting said host cell and/or surface with a modulator protein as described herein; and
(B) thereby preventing damage to said host cell and/or surface damage.

In the context of the method for preventing, the term "host cell" includes each and every living cell. Preferably, said cell is amenable to damage by components of the classical and/or lectin pathway of the complement system. Thus, the cell may in particular be an eukaryotic cell. Preferably, the cell is a cultured cell, i.e. preferably is cultured *in vitro,* preferably in a medium containing blood or a blood-derived product, such as serum.

In the context of the method for preventing, the term "surface" includes each and every surface which is known or suspected to be amenable to be damaged by components of the classical and/or lectin pathway of the complement system. Thus, the surface may be the surface of a cell layer or a surface of extracellular material produced by such a cell layer, may be a functional surface, e.g. of a bead or a biosensor, or may be a porous material, e.g. a filter such as a hemodialysis filter or any other biomaterial. Preferably the surface is planned or at risk of being contacted with a composition of matter containing blood or a blood-derived product, such as serum or other biofluids that have active complement components present such as cerebrospinal fluid (CSF) or synovial fluid (SF).

The present invention moreover relates to a device comprising the modulator protein described herein and/or the pharmaceutical composition described herein.

The term "device", as used herein relates to a system of means comprising at least the means described, preferably operatively linked to each other and/or further means such as to allow administration of the compound or of the composition specified. Preferred means for administering a modulator protein are well known in the art and are described herein above. How to link the means in an operating manner will depend on the type of means included into the device and on the kind of administration envisaged. Preferably, the means are comprised by a single device in such a case. Said device may accordingly include a delivery unit for the administration of the modulator protein and, optionally, a storage unit for storing said modulator protein until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the modulator protein. More preferably, the device is an intravenous infusion (IV) equipment comprising the modulator protein. Also preferably, the device is a tube or an endoscopic device comprising the modulator protein for flushing a site of administration, e.g. a heart, or further comprising a needle for topical application of the compound or composition.

The present invention also relates to a kit comprising the modulator protein described herein and/or the pharmaceutical composition described herein comprised in a housing.

The term "kit", as used herein, refers to a collection of the aforementioned compounds, means or reagents which may or may not be packaged together. The components of the kit may be comprised by separate vials (i.e. as a kit of separate parts) or provided in a single vial, e.g. as a composition as specified herein above. The housing of the kit in an embodiment allows translocation of the compounds of the kit, in particular common translocation; thus, the housing may in particular be a transportable container comprising all specified components. Moreover, it is to be understood that the kit of the present invention may be used for practicing the methods referred to herein above. It is preferably envisaged that all components are provided in a ready-to-use manner for practicing the methods referred to above. Further, the kit preferably contains instructions for carrying out said methods. The instructions can be provided by a user's manual on paper or in electronic form. For example, the manual may comprise instructions for interpreting the results obtained when carrying out the aforementioned methods using the kit. Preferably, the kit is adapted for use in a method of the present invention, more preferably is adapted to comprise all reagents required to perform said method or methods. Preferably, the kit comprises further components, e.g. a means of administration and/or a further inhibitor of complement activation.

The present invention also relates to a use of modulator protein as specified herein and/or the pharmaceutical composition as specified herein for inhibiting the classical and/or lectin pathway of complement activation on a host cell or a surface, wherein said use preferably is an *in vitro* use.

The present invention also relates to a method for detecting activation of the classical and/or lectin complement pathway in a sample, comprising (i) contacting said sample with a detection reagent comprising a binding module comprising, preferably consisting of, CCP domains 19 and 20 of a factor, bonded to a detector compound, and
(ii) detecting binding of the detection reagent to said sample, and thereby
(iii) detecting activation of the classical and/or lectin complement pathway in a sample.

The method for detecting activation, preferably, is an in vitro method.

In view of the above, the following embodiments are particularly envisaged:
Embodiment 1: A modulator protein comprising (i) a regulator module regulating the classical pathway of complement activation and/or the lectin pathway of complement activation and (ii) a binding module comprising, preferably consisting of, CCP domains 19 and 20 of a factor H.
Embodiment 2: The modulator protein of embodiment 1, wherein in the amino acid sequence of said binding module the amino acid sequence aspartate at position 1119 was exchanged for a glycine.
Embodiment 3: The modulator protein of embodiment 1 or 2, wherein said binding module comprises the amino acid sequence of SEQ ID NO: 1 or 2 or a sequence at least 70% identical thereto.
Embodiment 4: The modulator protein of any one of embodiments 1 to 3, wherein said binding module consists of the amino acid sequence of SEQ ID NO: 1 or 2 or a sequence at least 70% identical thereto.
Embodiment 5: The modulator protein of any one of embodiments 1 to 4, wherein said binding module comprises the amino acid sequence of SEQ ID NO:1 or 2.
Embodiment 6: The modulator protein of any one of embodiments 1 to 5, wherein said binding module consists of the amino acid sequence of SEQ ID NO:1 or 2.
Embodiment 7: The modulator protein of any one of embodiments 1 to 6, wherein said regulator module comprises a module of an effector protein of the classical and/or lectin pathway of complement activation, preferably a convertase decay accelerating module and/or a convertase or C4b (or C4b and C3b) specific cofactor activity module.
Embodiment 8: The modulator protein of any one of embodiments 1 to 7, wherein said regulator module comprises, preferably consists of an effector module of a C4b binding protein, of a decay accelerating protein, of a membrane cofactor protein, and/or of a complement receptor (CR).
Embodiment 9: The modulator protein of embodiment 8, wherein said C4b binding protein is C4b binding protein, wherein said decay accelerating protein is CD55, wherein said membrane cofactor protein is CD46, and/or wherein said CR is CR1.
Embodiment 10: The modulator protein of embodiment 8 or 9, wherein said regulator module comprises the amino acid sequence of at least one of SEQ ID NOs:3 and 8 to 12 or a sequence at least 70% identical thereto..
Embodiment 11: The modulator protein of any one of embodiments 1 to 10, wherein said regulator module comprises the amino acid sequence of SEQ ID NO:3 or a sequence at least 90% identical thereto.
Embodiment 12: The modulator protein of any one of embodiments 1 to 11, wherein said regulator module consists of the amino acid sequence of SEQ ID NO:3 or a sequence at least 70% identical thereto.
Embodiment 13: The modulator protein of any one of embodiments 1 to 12, wherein said regulator module comprises the amino acid sequence of SEQ ID NO:3.
Embodiment 14: The modulator protein of any one of embodiments 1 to 13, wherein said regulator module consists of the amino acid sequence of SEQ ID NO:3.
Embodiment 15: The modulator protein of any one of embodiments 1 to 14, wherein said modulator protein is a polypeptide.
Embodiment 16: The modulator protein of any one of embodiments 1 to 15, wherein said modulator protein comprises the amino acid sequence of SEQ ID NO:4 or 5, or a sequence at least 70% identical thereto.
Embodiment 17: The modulator protein of any one of embodiments 1 to 16, wherein said modulator protein consists of the amino acid sequence of SEQ ID NO:4 or 5, or a sequence at least 70% identical thereto.
Embodiment 18: The modulator protein of any one of embodiments 1 to 17, wherein said modulator protein comprises the amino acid sequence of SEQ ID NO:4 or 5.
Embodiment 19: The modulator protein of any one of embodiments 1 to 18, wherein said modulator protein consists of the amino acid sequence of SEQ ID NO:4 or 5.
Embodiment 20: The modulator protein of any one of embodiments 1 to 19, wherein said modulator protein binds to a C4b polypeptide, an iC4b polypeptide, a C4dg polypeptide, and/or a C4d polypeptide.
Embodiment 21: The modulator protein of any one of embodiments 1 to 20, wherein said modulator protein binds to a C4b polypeptide, an iC4b polypeptide, a C4dg polypeptide, and/or a C4d polypeptide, bound to a cell surface.
Embodiment 22: The modulator protein of any one of embodiments 1 to 21, wherein said modulator protein binds to cells on which the classical pathway and/or the lectin pathway of complement activation was activated.
Embodiment 23: The modulator protein of any one of embodiments 1 to 22, wherein said cells on which the classical pathway and/or lectin pathway of complement activation was activated are neurons, muscle cells, endothelial cells, cells in the kidney, endothelial cells, blood cells, or epithelial cells.
Embodiment 24: The modulator protein of any one of embodiments 1 to 23, wherein said modulator protein is comprised in a pharmaceutical composition.
Embodiment 25: The modulator protein of any one of embodiments 1 to 24, wherein said regulation is an inhibition.
Embodiment 26: A polynucleotide encoding the modulator protein of any one of embodiments 1 to 23, preferably encoding a polypeptide according to any one of embodiments 15 to 23.
Embodiment 27: A pharmaceutical composition comprising the modulator protein according to any one of embodiments 1 to 25 and/or the polynucleotide of embodiment 26.
Embodiment 28: The modulator protein according to any one of embodiments 1 to 25, the pharmaceutical composition according to embodiment 27 and/or the polynucleotide of embodiment 26, for use in medicine.
Embodiment 29: Use of the modulator protein according to any one of embodiments 1 to 25 and/or the polynucleotide of embodiment 26 for the manufacture of a medicament.
Embodiment 30: The modulator protein according to any one of embodiments 1 to 25, the pharmaceutical composition according to embodiment 27, and/or the polynucleotide of embodiment 26, for use in preventing and/or treating cell or host surface damage by components of the classical pathway and/or lectin pathway of complement activation.
Embodiment 31: The modulator protein according to any one of embodiments 1 to 25, the pharmaceutical composition according to embodiment 27, and/or the polynucleotide of embodiment 26, for use in preventing and/or treating a disease caused and/or aggravated by an activation of the classical and/or lectin pathway of complement activation.
Embodiment 32: Use of the modulator protein according to any one of embodiments 1 to 25 and/or the polynucleotide of embodiment 26 for the manufacture of a medicament for treating and/or preventing cell or host surface damage by components of the classical and/or lectin pathway of complement activation and/or for preventing and/or treating a disease caused and/or aggravated by an activation of the classical and/or lectin-dependent pathway of complement activation.
Embodiment 33: The subject matter of any one of embodiments 30 to 32, wherein said cell or host surface damage is tissue damage and/or organ damage.
Embodiment 34: The subject matter of any one of embodiments 31 to 33, wherein said disease is an infection by a pathogenic microorganism, systemic lupus erythematosus, lupus nephritis, antiphospholipid syndrome, catastrophic antiphospholipid syndrome, platelet transfusion refractoriness, IgA nephropathy, transplant-associated microangiopathy, hematopoetic stem cell transplantation-associated microangiopathy, antibody-mediated rejection, ischemia-reperfusion injury, secondary membranous nephropathy, xenotransplantation, acute kidney injury, or Guillain-Barre syndrome.
Embodiment 35: A method for treating and/or preventing cell damage by components of the classical and/or lectin pathway of complement activation and/or for preventing and/or treating a disease caused and/or aggravated by an activation of the classical and/or lectin-dependent pathway of complement activation in a subject, said method comprising
   (a) administering a modulator protein according to any one of embodiments 1 to 25 and/or the polynucleotide of embodiment 26 to said subject; and
   (b) thereby treating and/or preventing cell and/or host surface damage by components of the classical and/or lectin pathway of complement activation and/or for preventing and/or treating a disease caused and/or aggravated by an activation of the classical and/or lectin-dependent pathway of complement activation in said subject.
Embodiment 36: A method, preferably an in vitro method, for preventing damage by components of the classical and/or lectin pathway of complement activation to a host cell and/or surface, said method comprising
   (A) contacting said host cell and/or surface with a modulator protein according to any one of embodiments 1 to 25; and
   (B) thereby preventing damage to said host cell and/or surface damage.
Embodiment 37: A device comprising the modulator protein according to any one of embodiments 1 to 25, the pharmaceutical composition according to embodiment 27, and/or the polynucleotide of embodiment 26.
Embodiment 38: The device of embodiment 36, wherein said device is a means of administration.
Embodiment 39: A kit comprising the modulator protein according to any one of embodiments 1 to 25, the pharmaceutical composition according to embodiment 27, and/or the polynucleotide of embodiment 26, comprised in a housing.
Embodiment 40: The kit of embodiment 39, wherein said kit further comprises a further inhibitor of complement activation.
Embodiment 41: Use of a modulator protein according to any one of embodiments 1 to 25 and/or the pharmaceutical composition according to embodiment 27 for inhibiting the classical and/or lectin pathway of complement activation on a host cell or a surface.
Embodiment 42: The use of embodiment 41, wherein said use is an *in vitro* use.

All references cited in this specification are herewith incorporated by reference with respect to their entire disclosure content and the disclosure content specifically mentioned in this specification.

### Figure Legends

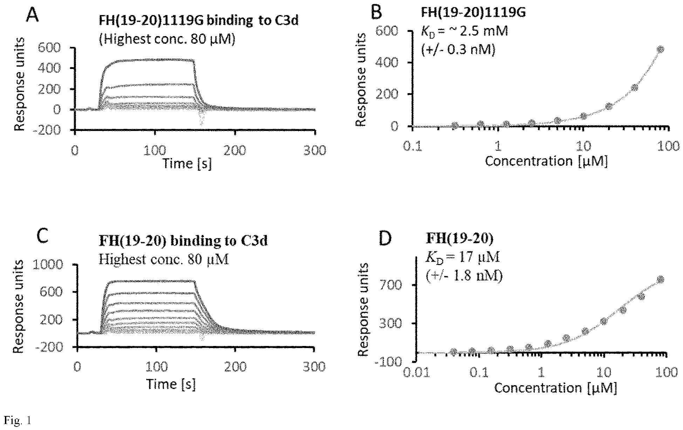

Fig. 1: SPR analysis of the binding affinity of the C-terminus of FH to C3d (cleavage product of AP). Here, 3000 response units (RU) of C3d were immobilized on a CMD500m sensor chip using amine coupling. The left side (A, C) shows the sensorgrams for injection of the corresponding factor H fragment (A: FH(19-20)1119G mutein, C: FH(19-20) wt) and the right side (B, D) shows the concentration-response curve with the dissociation constant (KD) determined by the 1:1 steady-state affinity model (B: FH(19-20)1119G mutein, D: FH(19-20) wt).

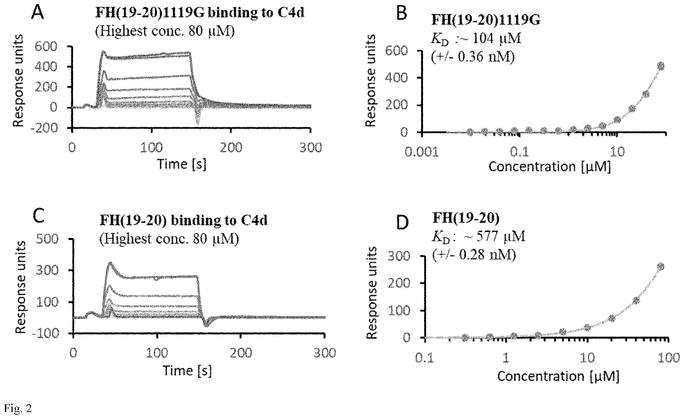

Fig. 2: SPR analysis of the binding affinity of the C-terminus of FH to C4d (cleavage product of CP). Here, 1100 response units (RU) of C4d were immobilized by amine coupling on a CMD500m sensor chip. The left side (A, C) shows the sensorgrams for injection of the corresponding factor H fragment (A: FH(19-20)1119G mutein, C: FH(19-20) wt) and the right side (B, D(shows the concentration-response curve with the dissociation constant (KD) determined by the 1:1 steady-state affinity model (B: FH(19-20)1119G mutein, D: FH(19-20) wt).

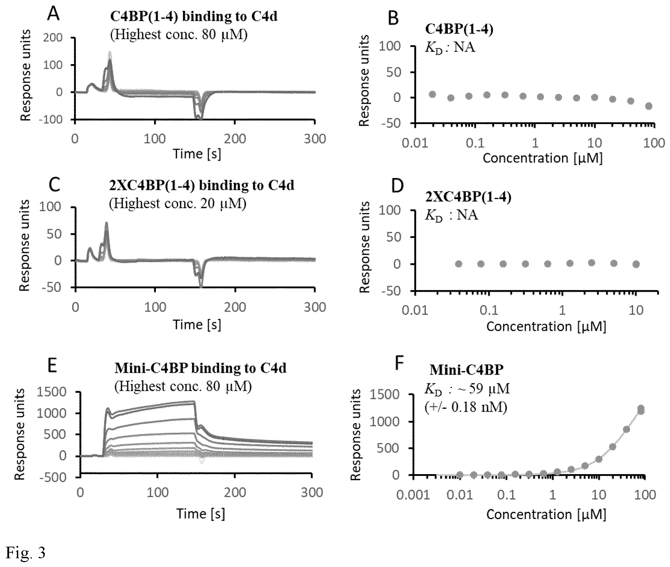

Fig. 3: SPR analysis of the binding affinity of fusion constructs with the C-terminus of FH to C4d (cleavage product of CP). Here, 1100 RU of C4d were immobilized on a CMD500m sensor chip using amine coupling. The left side (A, C, E) shows the sensorgrams for injection of the corresponding protein (A: C4BP(1-4), C: 2XC4BP, E: Mini C4BP) and the right side (B, D, F) shows the concentration-response curve with the dissociation constant (KD) determined by the 1:1 steady-state affinity model (B: C4BP(1-4), D: 2XC4BP, F: Mini C4BP). C4BP(1-4) = protein domains 1-4 of the C4-binding protein (regulator of CP); 2XC4BP(1-4) = same as C4BP(1-4), except that in this construct two copies of C4BP(1-4) are fused together; mini-C4BP= C4BP(1-4) fused to FH(19-20)1119G.

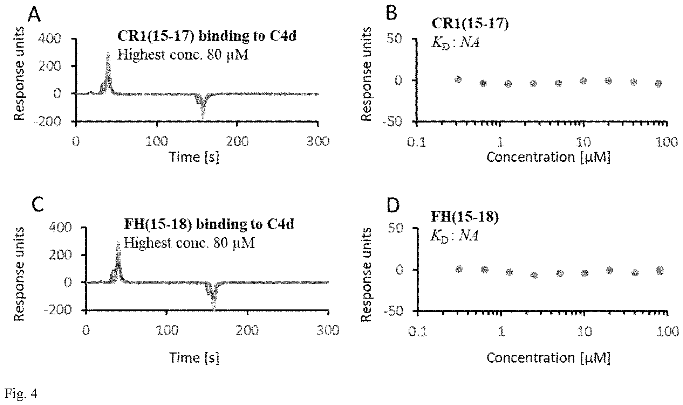

Fig. 4: SPR analysis of the binding affinity of control proteins to C4d (cleavage product of CP). Here, 487 RU of biotinylated C4d were immobilized on a streptavidin sensor chip. The left panels (A, C) show the sensorgrams for injection of the corresponding control protein and the concentration-response curve is shown on the right (B, D) with the dissociation constant (KD) determined by the 1:1 steady-state affinity model. CR1(15-17) = protein domains 15-17 of the complement regulator complement receptor 1 (A, B), which is active in all activation pathways of the complement system; FH(15-18) = protein domains 15-18 of FH (C, D), which has no particular known binding or regulatory activity.

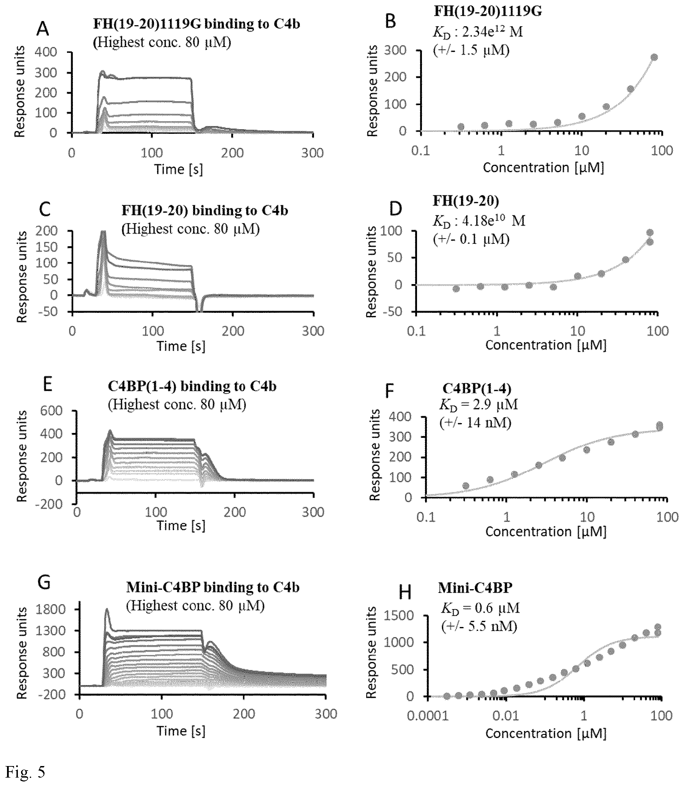

Fig. 5: SPR analysis of the binding affinity of fusion constructs with the C-terminus of FH to C4b (cleavage product of CP). Here, 2300 RU of biotinylated C4b were immobilized on a streptavidin sensor chip. The left side (A, C, E, G) shows the sensorgrams for injection of the corresponding protein (A: FH(19-20)1119G mutein; C:FH(19-20) wt; E: C4BP(1-4), G: MiniC4BP) and the right side (B, D, F, H) shows the concentration-response curve with the dissociation constant (KD) determined according to the 1:1 steady-state affinity model (B: FH(19-20)1119G mutein; D:FH(19-20) wt; F: C4BP(1-4), H: MiniC4BP).

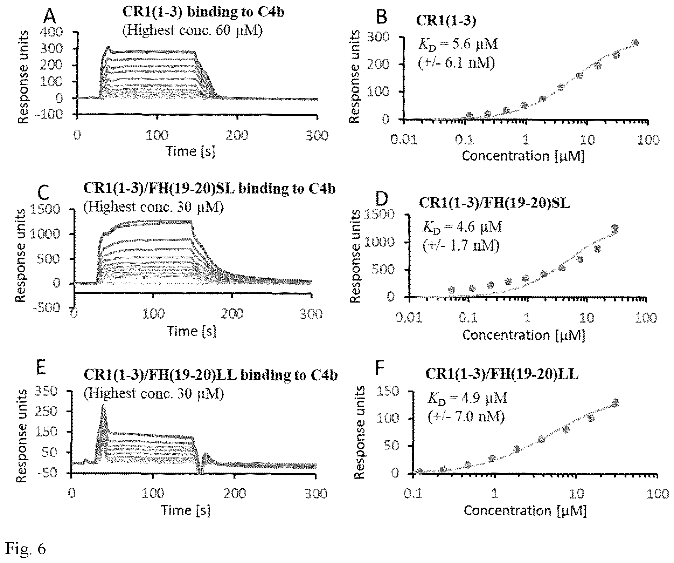

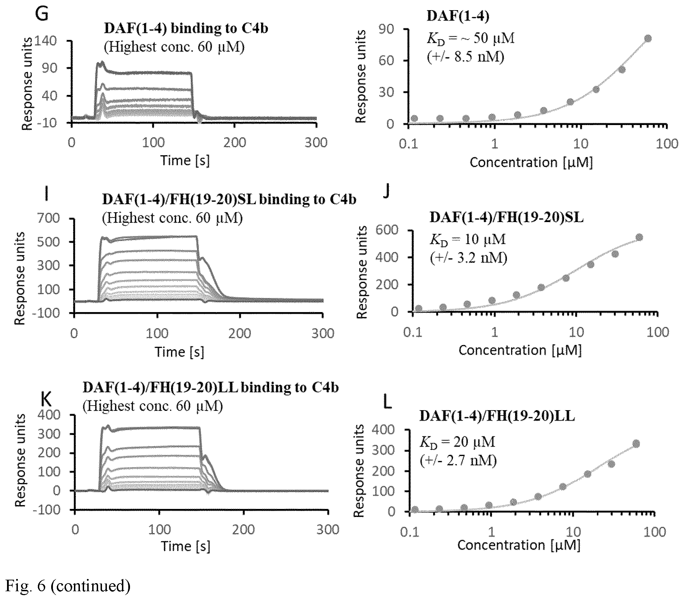

Fig. 6: SPR analysis of the binding affinity of fusion constructs with the C-terminus of FH to C4b (cleavage product of the CP). Here, 3127 RU of biotinylated C4b were immobilized on a streptavidin sensor chip. The left panels (A, C, E, G, I, K) show the sensorgrams for injection of the corresponding protein and the concentration-response curves are shown on the right (B, D, F, H, J, L) with the dissociation constant (KD) determined according to the 1:1 steady-state affinity model. CR1(1-3) = protein domains 1-3 of the complement regulator complement receptor 1 (A, B) which is active in all activation pathways of the complement system; CR1(1-3)/FH(19-20)SL = fusion of CR1 protein domains 1-3 and FH(19-20) with no additional linker in between (= SL) (C, D); CR1(1-3)/FH(19-20)LL = fusion of CR1 protein domains 1-3 and FH(19-20) with an additional glycine linker in between (= LL) (E, F); DAF(1-4) = protein domains 1-4 of the complement regulator decay-accelerating factor which is active in all activation pathways of the complement system (G, H); DAF(1-4)/FH(19-20)SL = fusion of DAF protein domains 1-4 and FH(19-20) without an additional linker in between (= SL) (I, J); DAF(1-4)/FH(19-20)LL = fusion of DAF protein domains 1-4 and FH(19-20) with an additional glycine linker in between (= LL) (K, L).

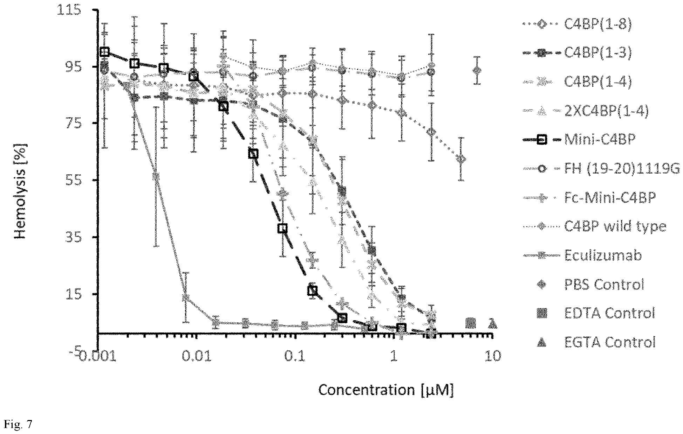

Fig. 7: Functional hemolysis test of fusion constructs with FH(19-20)1119G. In this hemolysis test, erythrocytes are washed and diluted to a defined cell density before being mixed with a defined percentage of serum +/- analyte and incubated. After incubation, the remaining cells are centrifuged, and the supernatant is measured at 405 nm. The absorbance gives an indication of how much hemoglobin was released and accordingly how many cells were lysed in % when the value is compared to a lysis control (complete, osmotic lysis in water). In this particular test, sheep erythrocytes (which are not lysed complement-mediated in human serum) were loaded with antibodies to specifically activate the CP. Samples in which the serum has been supplemented with EDTA (blocks any complement-mediated lysis), EGTA (blocks CP-mediated lysis) or PBS instead of inhibitor serve as controls. HA = hemolysis assay; NHS = normal human serum; Eculizumab = antibody approved for therapy of complement-mediated diseases that binds the terminal protein C5.

The following Examples shall merely illustrate the invention. They shall not be construed, whatsoever, to limit the scope of the invention.

### Example 1: Conditioning and immobilization of ligands in random orientation

A CMD500m chip was left at room temperature for 20-30 minutes prior to utilization and inserted into a Reichert SR7500DC system which was equilibrated in PBS-T (0.005% Tween20) as standard running buffer. The general flow rate was set to 25 µl/min. First, the chip was conditioned by five repetitive washes of 50 mM sodium hydroxide (NaOH) to expose the dextran and the attached carboxyl groups that are needed to react in the next step. The reactive groups then were activated via a 1:1 mix of 0.1 M sulfo-N-hydroxysulfosuccinimide (s-NHS/ sulpho-NHS) dissolved in water which results in more stable amine-reactive intermediates and higher coupling efficiency together with 0.1 M 1-ethyl-3-(3-dimethylamino-propyl) carbodiimide hydrochloride (EDC) that serves as zero-length crosslinking agent dissolved in 5 mM MES buffer at pH 6.0.

This reaction mixture activates the carboxyl groups of the carboxymethyldextran hydrogel on the CMD500m chip allowing for covalent binding of the activated carboxyl groups to the primary amine groups of the protein to be immobilized onto the chip. By this way the ligands were immobilized covalently. Immobilization of all ligands took place on the left flow cell only, while the right flow cell was activated without exposure to the ligands generating the reference flow cell. Finally, an additional quenching step with 1 M ethanolamine at pH 8.5 over both flow cells was performed to react with any remaining activated carboxyl groups. The immobilized units of the ligand are obtained by subtracting the signal of the reference chamber from the calculated signal of the immobilization chamber. After each ligand immobilization, a positive control test was done by injecting an analyte that is well known to bind the attached or immobilized ligand. Once a good quality binding signal was detected, the injection of the analytes of interest as a serial dilution was performed.

Exact concentration and buffer pH of each immobilized ligand is demonstrated below (Table 1).

**Table 1: Different ligands immobilized in random orientation using CMD500m sensorchip**

| **The immobilized Ligand** | **Its concentration and the used buffer** | **Immobilized RUs** |
|---|---|---|
| C4b (Comptech, USA) | Applied at 30 µg/ml in 5 mM Na-Acetate pH 4.5 | 2300 RUs |
| C4d (produced enzymatically) | Applied at 80 µg/ml in 5 mM Na-Acetate pH 4.0 | 3000 RUs |

### Example 2: Conditioning and immobilization of the ligands in "physiological orientation"

In this Example, the thioester moiety of the ligands C4b and C4d was biotinylated to bind covalently to the avidin surface of the sensor chip. C4b (Comptech, USA) and C4d was produced and biotinylated using an NHS-PEG4-Biotin Kit (Thermo Fisher Scientific, USA) according to manufacturer's instructions.

**Table 2: Different ligands immobilized in physiological orientation using SAP sensor chips; running buffer was PBS-T (0.005% Tween20)**

| **The immobilized Ligand** | **Its concentration and the used buffer** | **Immobilized RUs** |
|---|---|---|
| **Biotinylated C4b** | Applied 100 nM in running buffer | 2500 RUs |
| **Biotinylated C4d** | Applied 100 nM in running buffer | 500 RUs |
| **Biotinylated C3b** | Applied 100 nM in running buffer | 3260 RUs |

### Example 3: Binding program of different produced proteins

To assess the binding characteristics of different C4BP and other related proteins, a 1:2 (i.e. a 1+1) dilution series of each protein was prepared and injected starting from the lowest to the highest concentration. The highest concentration for each analyte was injected twice (duplicates) to show reproducibility of the experiment. Before analyte injection, the chip was washed twice with running buffer to produce baseline curves. At the end of each injection cycle, the residual bound molecules were forcefully eluted via repetitive injections of regeneration buffer (1 M NaCl). The response units were plotted against the analyte concentrations and the affinity was fitted to a 1:1 steady state affinity model resulting in KD-values given.

### Example 4: Classical pathway hemolysis assay using 5% NHS

Sheep erythrocytes (shRBCs) were washed three times with Dulbecco's phosphate-buffered saline (DPBS) to eliminate the storing solution (2500g, 3 min). Part of the cells was used to obtain an optical density of 1.3 at A405 nm when lysing 10 µl cells in 140 µl H₂O and measuring the absorbance of 100 µl lysate. Those cells were then diluted in PBS++ (1x PBS + 0.15 mM CaCh + 0.5 mM MgCh as a final assay concentration).

Afterwards, the shRBCs were sensitized by incubation with rabbit anti-sheep RBCs antibodies (hemolysin) (Rockland Immunochemicals, Inc., USA) diluted 1:40 in 5 mM PBSE. These sensitized cells were incubated 20 min at 37 °C. After the incubation time with hemolysin, the cells were washed twice in DPBS and resuspended in PBS++ to the same initial concentration (A405 determined value) and kept on ice until used in the assay. The human sera obtained from different healthy donors were prepared for the assay by a pre-absorption step; this means incubating the serum with similar volume of washed (shRBCs) (1:1) for 30 min on ice with intermittent shaking to make sure that all antibodies were pre-absorbed from the sera. Afterwards, the incubated sera were washed twice for 2 min at 2500 g. The supernatant (preabsorbed serum) was transferred and mixed with magnesium and calcium ions at a concentration of 0.5 mM and 0.15 mM, respectively. This is done to ensure efficient functionality of the CP, as calcium ions are important for the C1 complex function and magnesium ions are necessary for convertase formation. The tested proteins were prepared in serial dilutions (1:2) with DPBS.

After preparing each component of the assay (the diluted sera, the tested proteins and the shRBCs), all components were mixed in 96 well plates (Corning Inc., USA) so that each well had 80 µl diluted serum (i.e. + PBS + 0.15 mM CaCh + 0.5 mM MgCh; 5 % final serum content), 10 µl protein inhibitor solution and 10 µl A405 adjusted, sensitized cell. The diluted serum was pipetted first then the tested protein in serial dilution, both were mixed well and allowed to incubate for 30 min incubation (on ice) prior to addition of the sensitized shRBCs which were added last. To stop the reaction, 50 µl of ice-cold PBS/EDTA (50 mM) were added to each well. Afterwards the plate was centrifuged at 1500g for 3 min and 100 µl supernatant from each well was transferred carefully into a fresh flat bottom 96 well plate (BRANDplates^{®}, 96-well, BRAND GmbH, Germany) for absorbance measurement (405 nm). For quality control of each experiment, a set of blanks and controls were prepared. Serum blank was prepared by mixing 80 µl diluted serum with 20 µl DPBS, a positive control or full lysis was performed by pipetting 90 µl deionized water (osmotic lysis) with 10 µl cells. Another positive control called PBS control was done by incubating 80 µl serum mixture with 10 µl PBS and 10 µl sensitized cells without any inhibitor (complete lysis expected). Additionally, PBS/EDTA 10 mM (shortly called EDTA 10 mM control) was included by mixing 80 µl of serum mixture with 10 µl of 100 mM EDTA plus 10 µl sensitized shRBCs where the EDTA should chelate all the calcium and magnesium ions and hence hinder the cells lysis. The last control was the PBS/EGTA 10 mM (shortly called EGTA 10 mM control) that was prepared by pipetting 80 µl of serum mixture with 10 µl of 100 mM EGTA plus 10 µl of sensitized shRBCs. In all hemolysis assays, for each analyte a duplicate was done, and each assay was performed at least for three repetitions.

### Example 5: FH(19-20) binding to C3d (control Example)

Factor H (FH) is a regulator of the alternative pathway of the complement system (= AP) and therefore binds proteins of the AP as expected and frequently described in the literature. Figure 1 shows results of an experiment in which the binding affinity was measured by surface plasmon resonance spectroscopy (SPR). For this, the cleavage product C3d was immobilized and the recombinantly produced FH fragment consisting of the last two protein domains (= FH(19-20)) was injected onto the immobilized C3d and the binding signal was measured. In addition, a mutation variant of this protein fragment is seen, in which the amino acid aspartic acid has been exchanged for glycine at position 1119 in FH. This mutation has been described to significantly reduce binding to C3b or C3d (C3d is a fragment of C3b).

### Example 6: FH(19-20) binding to C4d

Repeating Example 5 with C4d (cleavage product of the classical activation pathway (CP)) instead of C3d as a bait unexpectedly shows a concentration-dependent binding signal (see Figure 2).

In both cases, the dissociation constant cannot be determined precisely because the interaction is comparatively weak. The affinity values given are only approximations, since it was not possible to investigate sufficiently high concentrations to achieve an exact determination of the affinity.

FH(19-20) or the mutant FH(19-20)1119G was fused to regulatory active sections of different regulators of the complement system and again determined the affinities of the fusion constructs or the regulatory active sections (see Figure 3). The experiment clearly shows that C4BP(1-4) or two copies of it (2XC4BP) do not bind to C4d. In contrast, mini-C4BP (= fusion of C4BP(1-4) and FH(19-20)1119G) binds to C4d in a concentration-dependent manner.

### Example 7: Control Example

Repeating Example 6 with other control proteins, which are also protein segments of complement regulators, do not show concentration-dependent binding to C4d (see Figure 4).

### Example 8: FH(19-20) binding to C4b

FH(19-20) also binds to C4b (activation product of the classical pathway (CP) of the complement cascade), that results in C4d after further degradation), again confirming binding to C4d. Figure 5 shows that FH(19-20)1119G by itself binds exceptionally weakly to C4b, but the increase in affinity from ~ 2.9 µM for C4BP(1-4) alone to 0.6 µM for the combination of C4BP(1-4) and FH(19-20)1119G (= mini-C4BP) demonstrates that FH(19-20)1119G increases the initial affinity of C4BP(1-4) via protein fusion.

This is a general effect, since the fusion of FH(19-20) with regulators other than C4BP also increases the affinity of the regulator for C4b (see Figure 6). The varies for different regulators and also depends on the linker (or its length) that is in the fusion between the regulator and FH(19-20).

### Example 9: Complement inhibition

This positive effect can be used to target fusion constructs such as mini-C4BP to surfaces that have to be protected from CP activation or have recently suffered damage from CP activation and are therefore occupied by C4d. In functional cell assays with erythrocytes, we demonstrated that the protective effect of the CP regulator C4BP(1-4) is enhanced by fusion with FH(19-20)1119G (see Figure 7). In this experiment, C4BP(1-4) alone achieved an IC50 of 0.59 µM and in fusion with FH(19-20)1119G (= mini-C4BP) an IC50 of 0.06 µM. This result shows and impressively proves that the cell-protective effect was enhanced (by a factor of 10), although FH(19-20)1119G does not have a high affinity to the CP cleavage product C4d.

### References

- Hillmen et al. (2006), NEJM355(12):1233
- Parkin & Cohen (2001) The Lancet 357: 1777-89
- Ricklin et al. (2010) Nature Immunology 11: 785-797
- Ricklin et al (2017), Mol Immunol. 89:10-21
- Romay-Penabad et al (2014), Lupus 23(12):1324
- Schmidt et al. (2008), Clin Exp Immunol.151(1):14-24
- WO 2013/142362 A1

## Claims

1. A modulator protein comprising (i) a regulator module regulating the classical pathway of complement activation and/or the lectin pathway of complement activation and (ii) a binding module comprising, preferably consisting of, CCP domains 19 and 20 of a factor H; for use in preventing and/or treating a disease caused and/or aggravated by an activation of the classical and/or lectin pathway of complement activation.

2. The modulator protein for use of claim 1, wherein said binding module comprises the amino acid sequence of SEQ ID NO:1 or a sequence at least 70% identical thereto.

3. The modulator protein for use of claim 1 or 2, wherein in the amino acid sequence of said binding module the amino acid sequence aspartate at position 1119 was exchanged for a glycine.

4. The modulator protein for use of any one of claims 1 to 3, wherein said regulator module comprises a module of an effector protein of the classical and/or lectin pathway of complement activation, preferably a convertase decay accelerating module and/or a specific module that has cofactor activity for proteolytic cleavage of C4b, or C4b and C3b, by factor I.

5. The modulator protein for use of any one of claims 1 to 4, wherein said regulator module comprises, preferably consists of, an effector module of a C4b binding protein, of a decay accelerating protein, of a membrane cofactor protein, and/or of a complement receptor (CR).

6. The modulator protein for use of any one of claims 1 to 5, wherein said C4b binding protein is C4b binding protein, wherein said decay accelerating protein is CD55, wherein said membrane cofactor protein is CD46, and/or wherein said CR is CR1.

7. The modulator protein for use of any one of claims 1 to 6, wherein said regulator module comprises the amino acid sequence of at least one of SEQ ID NOs:3 and 8 to 12 or a sequence at least 70% identical thereto.

8. The modulator protein for use of any one of claims 1 to 7, wherein said regulator module comprises the amino acid sequence of SEQ ID NO:3 or a sequence at least 70% identical thereto.

9. The modulator protein for use of any one of claims 1 to 8, wherein said modulator protein is a polypeptide.

10. The modulator protein for use of any one of claims 1 to 9, wherein said modulator protein comprises the amino acid sequence of SEQ ID NOs:4 or 5, or a sequence at least 70% identical thereto.

11. The modulator protein for use of any one of claims 1 to 10, wherein said disease is an infection by a pathogenic microorganism, systemic lupus erythematosus, lupus nephritis, antiphospholipid syndrome, catastrophic antiphospholipid syndrome, platelet transfusion refractoriness, IgA nephropathy, transplant-associated microangiopathy, hematopoetic stem cell transplantation-associated microangiopathy, antibody-mediated rejection, ischemia-reperfusion injury, secondary membranous nephropathy, xenotransplantation, acute kidney injury, or Guillain-Barre syndrome.

12. A modulator protein comprising (i) a regulator module regulating the classical pathway of complement activation and/or the lectin pathway of complement activation and (ii) a binding module comprising, preferably consisting of, CCP domains 19 and 20 of a factor H.

13. The modulator protein of claim 12, wherein said modulator protein is a modulator protein as specified in any one of claims 1 to 11.

14. A kit comprising the modulator protein according claims 12 or 13 comprised in a housing.

15. A method, preferably an in vitro method, for preventing damage by components of the classical and/or lectin pathway of complement activation to a host cell and/or surface, said method comprising
(A) contacting said host cell and/or surface with a modulator protein according to claim 12 or 13; and
(B) thereby preventing damage to said host cell and/or surface damage.
